# EUROPEAN PATENT SPECIFICATION

(11) **EP 1 060 757 B1**
(45) Date of publication and mention of the grant of the patent: **06.09.2006**
(21) Application number: 00650055.7
(22) Date of filing: 19.05.2000
(51) Int. Cl.: A61M 25/00, F16L 3/223, F16L 3/237

(54) **A coiling clip for a catheter guide wire**
Klemme zum Aufrollen eines Katheter Führungsdrahtes
Attache pour enrouler un fil de guidage de cathéter

(30) Priority: 19.05.1999 US 314706
(43) Date of publication of application: 20.12.2000
(73) Proprietor: Seacrest Technology Limited, Galway, County Galway (IE)
(72) Inventor: BYRNES, Raymond, Rhode Island 02816 (US); DI PETRILLO, Robert, Rhode Island 02882 (US)
(74) Representative: Schütte, Gearoid

(56) References cited:
- EP-A- 0 587 984
- EP-A- 0 720 836
- EP-A- 0 782 868
- US-A- 4 406 042
- US-A- 5 843 002

## Description

There are many medical procedures where catheters are used. Frequently, other apparatus is used with the catheter. For example, when a catheter is introduced into a patient's blood vessel such as an artery or vein, guide wires are routinely used.

In a typical procedure, one uses the guide wires for the positioning of a catheter. For instances, in the Seldinger technique, a catheter introducer is used which has a relatively short flexible cannula which is placed within the patient's blood vessel. Actual insertion of the cannula is assisted by the use of a needle which is positioned within the cannula and is thus inserted into the blood vessel. Upon insertion, the needle is withdrawn, leaving the cannula tip within the blood vessel while the body of the catheter introducer remains external of the patient.

A guide wire is then inserted through the catheter introducer and is extended through the tip of the cannula within the patient's blood vessel until it is positioned with its tip at the desired location within the patient. Upon removal of the catheter introducer, the guide wire remains in the patient and a long catheter is easily slid over the guide wire to the desired position and the guide wire withdrawn. Thus, the catheter remains within the patient having its distal end located at the proper position within the patient's blood vessel.

These catheters can be used for a variety of medical techniques including angioplasty, gene delivery, etc. Thus, the guide wires, once introduced into the body, can extend to distant sites.

Anytime any object is introduced into the blood stream, care must be taken to ensure that sterility is maintained and that the object used does not cause infection. One method by which this goal has been met is the trend to single use devices which are sterilised when made, then shipped and ready to use.

Such guide wires are delivered through normal shipping channels and are subject to considerable handling prior to and during shipment. The guide wire itself is packaged within a dispenser or protective tube in a coiled form referred to as a "spiral wound" dispenser. A typical guide wire introducer is disclosed in U.S. Patent No. 5,282,479. In EP 0782868 there is shown packaging for a stent delivery catheter in which the catheter is wound in a coil and retained in the coiled position by clamps having juxtaposed channels for reception of overlapping coils of the catheter. In US 4406042 there is disclosed a tubing clip having a pair of channels each having a narrowed neck at one end to crimp and grip a tube passing therethrough. In EP 0720836 there is disclosed an attachment clip for surgical tubes and cables. The clip has at least one opening with ribs designed to slidably secure the tube or cable.

Presently, several clips or retainers referred to as "coiling clips" are used to anchor the coiled tube. These are devices having a smooth inner surface which hold the tube by exerting pressure against the tube wall. Such a coiling clip comprises at least two side-by-side C-shaped sockets having longitudinal slit-like socket mouths for reception of the tube. However, during transport, it is common for a segment of the tube to "pop out" of the clip. Thus, before using the device, the physician must waste valuable time inserting the tube back into the clip. Such additional handling can not only waste time, but may compromise the sterility. This can also cause a serious problem for the physician if, when using the dispenser, a portion of the tube pops out of the clip. Accordingly, there is a need for a coiling clip that more securely anchors the spiral wound dispenser.

### Statements of Invention

According to the invention, there is provided a guide wire assembly, including at least one plastics coiling clip and a dispenser tube housing a catheter wire, the dispenser tube wound into a coil on the coiling clip, the coiling clip comprising at least two side-by-side resilient C-shaped sockets having longitudinal slit-like socket mouths for reception of the tube, each socket having a tube supporting and contacting internal bearing surface as defined in claim 1.

In one embodiment the socket mouths are in side-to-side relationship.

In another embodiment adjacent socket mouths are offset around the clip.

Ideally, the coiling clip is of a plastics material.

In another embodiment the dispenser tube has a plurality of longitudinal grooves for interengagement with the protuberance forming ribs.

Preferably, the coiling clip is of a harder material than that of the dispenser tube such that the coiling clip protuberances form indentations in the dispenser tube.

### Detailed Description of the Invention

The invention will be more clearly understood from the following description of some embodiments thereof, given by way of example only, with reference to the accompanying drawings, in which:-
Fig. 1 is a perspective view of a coiling clip of the present invention,
Fig. 2 is a perspective view of the coiling clip of Fig. 1 engaging a dispenser tube,
Fig. 3 is a perspective view of a guide wire assembly incorporating some of the coiling clips of Fig. 1,
Fig. 4 is a perspective view of an alternative construction of dispenser tube engaging the coiling clip of Fig. 1,
Fig. 5 is a sectional view along the lines V-V of Fig. 4,
Fig. 6 is a perspective view of another construction of coiling clip, which does not fall under claim 1,
Fig. 7 is a perspective view of a further construction of coiling clip,
Fig. 8 is a perspective view of another coiling clip, and
Fig. 9 is a perspective view of a further coiling clip according to the invention.

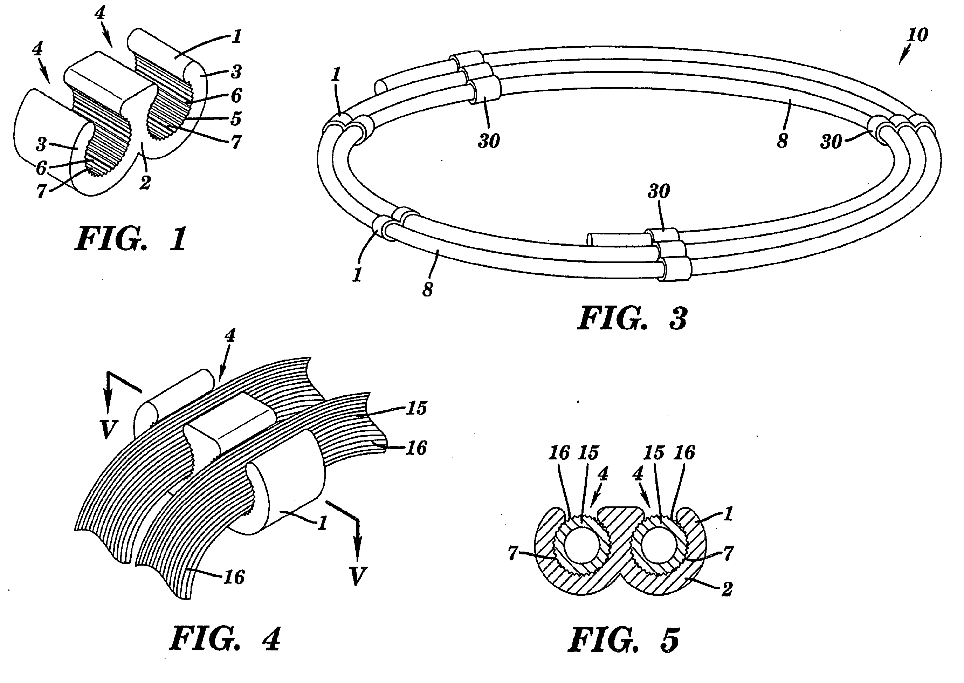

Referring to the drawings and initially to Figs. 1 to 3 thereof, there is provided a coiling clip, indicated generally by the reference numeral 1, having a body 2 mounting two side-by-side resilient C-shaped sockets 3, each having a longitudinal slit-like socket mouth 4. Each socket 3 has a tube supporting and contacting internal bearing surface 5 for reception of a dispenser tube. The bearing surface 5 carries a number of protuberances 6, in this embodiment formed by longitudinally arranged parallel striations or ribs 7.

Referring now to Fig. 2, there is illustrated, by interrupted lines, portion of a dispenser tube 8 mounted in the coiling clip 1.

Referring now specifically to Fig. 3, to form a guide wire assembly 10 from a coiling clip 1 and a dispenser tube 8 which dispenser tube 8 houses a catheter wire, the coiling clip 1 is taken and the dispenser tube 8 is inserted into the clip 1. Then, the dispenser tube 8 is wound into a coil of the desired size and again, the dispenser tube 8 is inserted in the clip 1 to form and hold the coil in position. Then, additional clips 1 are used and the dispenser tube 8 is inserted into them to provide the guide wire assembly, indicated clearly in Fig. 3 and identified by the reference numeral 10. It will be noted that the guide wire assembly holds other clips, identified by the reference numeral 30, which have not yet been described but will be described later.

It has been found that a turntable is particularly suitable for the forming of the guide wire assembly 10. If a turntable is used, the turntable may be either a driven turntable or simply rotated by the operators hands. If a turntable is used, then a number of clips 1 are placed on the turntable and then the tube 8 is inserted into one of the clips 1, the turntable is rotated slightly, the tube is inserted into the next clip on the turntable by pushing the tube 8 into the socket mouths of each clip. This is continued as the turntable is rotated thus progressively inserting the tube 8 into the socket mouths 4 of the clips 1. This causes the tube 8 to be wound into a coil of the desired size and when this is finished, the assembly 10 is formed. Generally, all that happens is that one keeps on winding or rotating the turntable and inserting the tube 8 into the clips until all the assembly is formed. In some embodiments, not necessarily all the clips will be immediately inserted onto the tube 8 as the coil is formed. Some may be inserted after the coil has been formed since all that is required from the clips is that there be sufficient clips on the turntable to retain the coil in position.

A test was carried out on the clip of Fig. 1. A four inch (10.16 cm) length of standard 0.152 inches (0.386 cm) tubing having a smooth outer surface was reinforced with a length of metal located on the inside of the tubing was provided. This tube was located on a looped chain at both ends. The chains were attached to a tensile force tester. The clip being tested was clamped to the bottom section of the tensile tester and the speed rate was set at 50 rpm. The test was carried out against a standard clip of the prior art. The following results were obtained:

| Sample No. | Existing Clip 51001 | New Clip 51115 |
|---|---|---|
| 1 | 0.8 lbs | 1.8 lbs |
| 2 | 0.7 lbs | 2.0 lbs |
| 3 | 0.8 lbs | 1.9 lbs |
| 4 | 0.8 lbs | 2.1 lbs |
| 5 | 0.8 lbs | 1.8 lbs |
| 6 | 0.8 lbs | 2.0 lbs |
| 7 | 0.7 lbs | 2.0 lbs |
| 8 | 0.8 lbs | 1.8 lbs |
| 9 | 0.7 lbs | 2.0 lbs |
| 10 | 0.8 lbs | 1.8 lbs |

As can be seen there is a significantly greater resistance to movement and a much higher tensile value when removed from the clip. This clearly indicated that the clip according to the invention has considerably tighter grip than that of the prior art.

Referring now to Figs. 4 and 5, there is illustrated an alternative construction of dispenser tube, identified by the reference numeral 15, again mounted within a coiling clip 1. In this embodiment, the dispenser tube 15 has a plurality of longitudinally arranged grooves 16 for reception of the ribs 7. These grooves 16 ideally are complementary to the ribs 7 such that the ribs 7 and grooves 16 engage or mesh, in effect, locking the tube in position as can be clearly seen from Fig. 5.

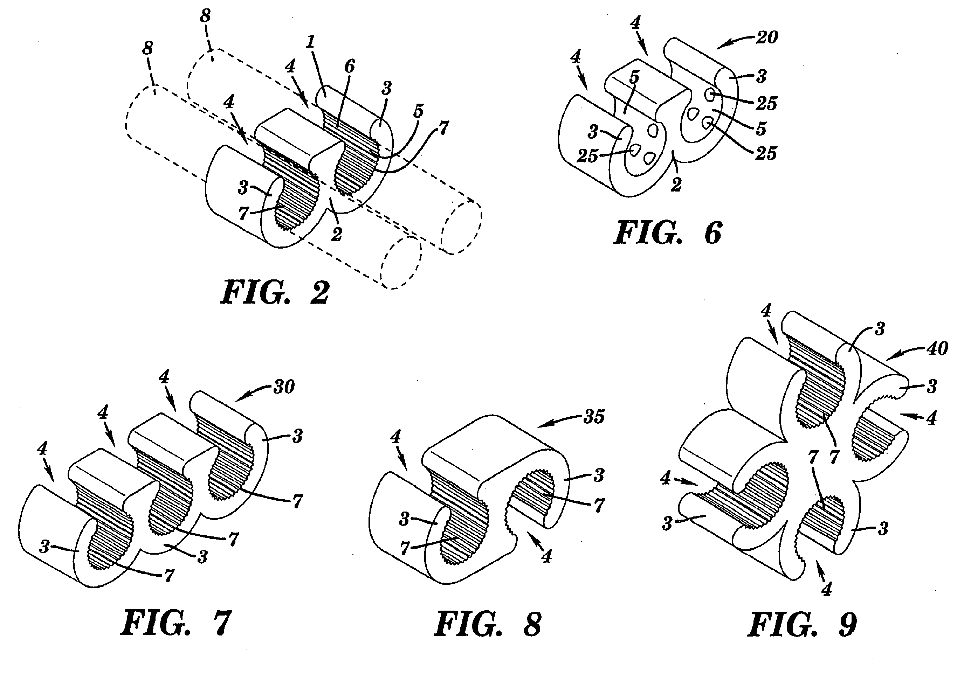

Referring to Fig. 7, there is illustrated an alternative construction of clip, indicated generally by the reference numeral 30, in which parts similar to those described with reference to Fig. 1 are identified by the same reference numerals. In this embodiment, there are three side-by-side sockets 3. These clips are used, as can be seen in Fig. 3, in forming the guide wire assembly 10. They are, in every respect, similar to the clips 1 of Fig. 1.

Fig. 8 illustrates an alternative construction of clip, indicated generally by the reference numeral 35, and again parts similar to those described with reference to Fig. 1 are identified by the same reference numerals. In this embodiment, adjacent socket mouths 4 are offset by 180°.

Referring to Fig. 9, there is illustrated a still further construction of clip, indicated generally by the reference numeral 40. Again, parts similar to those described with reference to the previous drawings are identified by the same reference numerals. In this embodiment, there are four sockets 3, all of which have their socket mouth offset around the clip 40.

It is envisaged that the clips and dispenser tube according to the present invention will be manufactured from a suitable plastics material. Ideally, the coiling clips should be made from a suitable material which is resilient and flexible and will withstand all sterilisation methods. Such materials include High Density Polyethylene (HDPE), Low Density Polyethylene (LDPE), Polypropylene (PP), Poly Vinyl Chloride (PVC), Polycarbonate or any other Polyolefin material used for extrusion or injection moulding, including various blends. A particularly suitable material is one formed from approximately 60% HDPE and 40% LDPE blended together. The clip can be formed using an extrusion process or can be injection moulded. Extrusion is preferred.

It is envisaged that while the protective tube may be manufactured from a plastics material, that the coiling clip is manufactured from a stronger or harder plastics material such that the protuberances on the coiling clip will engage into the dispenser tube so as to further lock the dispenser tube into the coiling dip.

Obviously, the dimensions of the clip have to be so formed as to provide a sufficient locking force onto the tube. Ideally, there is an inward pressure exerted by the coiling clip bearing surface on the outer surface of the tube. The idea of the invention is to ensure that the resilience of the clip, together with the projections, will be such as to lock the protective tube securely within the clip.

As has been shown by various tests, there has been a considerable increase in the resistance to pull out of a clip according to the present invention. Thus, there is less likely to be disassembly during transport.

Further it has been found that the clip slip has been reduced particularly when combined with the dispenser tube according to the invention. When clips slip they can cause difficulties when the assembly is inserted into certain proprietary procedural trays.

In the specification the terms "comprise, comprises, comprised and comprising" or any variation thereof and the terms "include, includes, included and including" or any variation thereof are considered to be totally interchangeable and they should all be afforded the widest possible interpretation.

The invention is not limited to the embodiments hereinbefore described but may be varied in both construction and detail within the scope of the claims.

## Claims

1. A guide wire assembly (10), including at least one plastics coiling clip (1) and a dispenser tube (8) housing a catheter wire, the dispenser tube(8) wound into a coil on the coiling clip (1), the coiling clip comprising at least two side-by-side resilient C-shaped sockets (3) having longitudinal slit-like socket mouths (4) for reception of the tube (8), each socket (3) having a tube supporting and contacting internal bearing surface (5), each bearing surface (5) including a plurality of upstanding tube engaging protuberances (6) dimensioned such as to cause a force-fit of the tube within the socket, the protuberances (6) comprising longitudinally arranged adjoining parallel striations or ribs (7) arranged side by side on the internal bearing surface (5), the resilience of the clip (1) together with the ribs (7) being such as to lock the dispenser tube (8) securely within the clip (1).

2. A guide wire assembly as claimed in claim 1, in which the socket mouths (4) are in side-by-side relationship.

3. A guide wire assembly as claimed in claim 1, in which adjacent socket mouths (4) are offset around the clip (35, 40).

4. A guide wire assembly as claimed in any preceding claim, in which the dispenser tube (15) has a plurality of longitudinal grooves (16) for interengagement with the protuberance forming ribs (7).

5. A guide wire assembly as claimed in any preceding claim, in which the coiling clip (1, 20, 30, 35, 40) is of a harder material than that of the dispenser tube (8, 15) such that the coiling clip protuberances form indentations in the dispenser tube.

## Patentansprüche

1. Führungsdrahtbaugruppe (10), die mindestens eine Kunststoffwicklungsklammer (1) sowie ein Spenderohr (8), das einen Katheterdraht beherbergt, umfasst, wobei das Spenderohr (8) an der Wicklungsklammer (1) zu einer Wicklung gewickelt ist, die Wicklungsklammer mindestens zwei nebeneinander angeordnete elastische C-förmige Aufnahmeelemente (3) umfasst, die längliche schlitzartige Öffnungen (4) des Aufnahmeelements zur Aufnahme des Rohrs (8) aufweisen, jedes Aufnahmeelement (3) eine Rohr stützende und berührende innere Auflagefläche (5) aufweist, jede Auflagefläche (5) eine Vielzahl aufrecht stehender Vorsprünge (6) umfasst, die in das Rohr eingreifen und so bemessen sind, dass ein Klemmsitz des Rohrs in dem Aufnahmeelement verursacht wird, die Vorsprünge (6) in Längsrichtung angeordnete benachbarte parallele Riffelungen oder Rippen (7) umfassen, die nebeneinander an der inneren Auflagefläche (5) angeordnet sind, und die Elastizität der Klammer (1) zusammen mit den Rippen (7) so beschaffen ist, dass das Spenderohr (8) fest in der Klammer (1) verriegelt wird.

2. Führungsdrahtbaugruppe nach Anspruch 1, wobei die Öffnungen (4) des Aufnahmeelements in einem nebeneinander angeordneten Verhältnis vorgesehen sind.

3. Führungsdrahtbaugruppe nach Anspruch 1, wobei benachbarte Öffnungen (4) des Aufnahmeelements um die Klammer (35, 40) herum versetzt angeordnet sind.

4. Führungsdrahtbaugruppe nach einem der vorhergehenden Ansprüche, wobei das Spenderohr (15) eine Vielzahl länglicher Rillen (16) für einen Eingriff in die Rippen (7), die einen Vorsprung bilden, aufweist.

5. Führungsdrahtbaugruppe nach einem der vorhergehenden Ansprüche, wobei die Wicklungsklammer (1, 20, 30, 35, 40) aus einem härteren Material besteht als das des Spenderohrs (8, 15), so dass die Vorsprünge der Wicklungsklammer in dem Spenderohr Einkerbungen bilden.

## Revendications

1. Ensemble de fil de guidage (10), comprenant au moins une attache à enrouler en matière plastique (1) et un tube distributeur (8) abritant un fil de cathéter, le tube distributeur (8) enroulé en une spirale sur l'attache à enrouler (1), l'attache à enrouler comportant au moins deux douilles élastiques juxtaposées en forme de C (3) ayant des bouches de douille longitudinales similaires à une fente (4) destinées à recevoir le tube (8), chaque douille (3) ayant une surface d'appui interne (5) qui porte un tube et qui est en contact avec celui-ci, chaque surface d'appui (5) comprenant une pluralité de protubérances verticales d'accrochage de tube (6) dimensionnées de telle manière à provoquer un ajustement serré du tube à l'intérieur de la douille, les protubérances (6) comportant des stries ou nervures parallèles contiguës arrangées de manière longitudinale (7) arrangées de manière juxtaposée sur la surface d'appui interne (5), l'élasticité de l'attache (1) de concert avec les nervures (7) étant telles que le tube distributeur (8) est fermement verrouillé à l'intérieur de l'attache (1).

2. Ensemble de fil de guidage selon la revendication 1, dans lequel les bouches (4) des douilles sont en relation juxtaposée.

3. Ensemble de fil de guidage selon la revendication 1, dans lequel les bouches (4) des douilles adjacentes sont décalées autour de l'attache (35, 40).

4. Ensemble de fil de guidage selon l'une quelconque des revendications précédentes, dans lequel le tube distributeur (15) a une pluralité de rainures longitudinales (16) à des fins d'enclenchement réciproque avec les nervures (7) formant les protubérances.

5. Ensemble de fil de guidage selon l'une quelconque des revendications précédentes, dans lequel l'attache à enrouler (1, 20, 30, 35, 40) est d'un matériau plus dur que celui du tube distributeur (8, 15) de telle manière que les protubérances de l'attache à enrouler forment des indentations dans le tube distributeur.
